(19) **Europäisches Patentamt**
**European Patent Office**
**Office européen des brevets**

(11) **EP 4 075 134 A1**

(12) **EUROPEAN PATENT APPLICATION**
published in accordance with Art. 153(4) EPC

(43) Date of publication:
**19.10.2022 Bulletin 2022/42**

(21) Application number: **20903562.5**

(22) Date of filing: **16.11.2020**

(51) International Patent Classification (IPC):
**G01N 33/543** (2006.01)      **G01N 33/68** (2006.01)

(86) International application number:
**PCT/CN2020/128990**

(87) International publication number:
**WO 2021/120943 (24.06.2021 Gazette 2021/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.12.2019 CN 201911320675**

(71) Applicant: **Resun (Shenzhen) Tech Co., Ltd.**
**Shenzhen, Guangdong 518110 (CN)**

(72) Inventors:
• **LIU, Ke**
  **Shenzhen, Guangdong 518110 (CN)**
• **XIONG, Gui**
  **Shenzhen, Guangdong 518110 (CN)**
• **WANG, Zhe**
  **Shenzhen, Guangdong 518110 (CN)**

(74) Representative: **Manitz Finsterwald**
**Patent- und Rechtsanwaltspartnerschaft mbB**
**Martin-Greif-Strasse 1**
**80336 München (DE)**

(54) **METHOD FOR DETECTING MICROPROTEINS IN SAMPLE SYSTEM**

(57)     A method for detecting a trace protein in a sample system, comprising: providing a primary antibody-modified immunomagnetic bead and a secondary antibody-modified nanoparticle for a to-be-detected protein; mixing and incubating the primary antibody-modified immunomagnetic bead and the secondary antibody-modified nanoparticle with a sample system containing the to-be-detected protein, so as to form a complex having a double-antibody sandwich structure; and allowing the complex to pass through a micro-nano pore device in the form of a single particle and trigger an electrical pulse signal, analyzing the electrical pulse signal to obtain a charge state or a volume/mass state of the complex, and calculating, according to the charge state or the volume/mass state, the amount of the to-be-detected protein in the sample system. In the method of the present application, an immunospecifically binding magnetic bead is used, a charge state of a single magnetic bead complex is detected, and an absolute amount of the trace protein is calculated. The method has a detection limit of down to a single protein molecule, can detect a trace protein at an amount below a lower limit of detection of conventional immunological detections, and can be widely used in immunological detection, microorganism detection, and cell separation, among other fields.

Fig. 1

Single-protein complex

## Description

## TECHNICAL FIELD

The present application relates to the technical field of protein detection, in particular to a method for detecting a trace protein in a sample system.

## BACKGROUND

Enzyme-linked immunosorbent assay is presently the most widely used immunological detection method among several commonly used immunological detection techniques. The method, by means of labeling a secondary antibody with an enzyme, combines the specificity of an antigen-antibody reaction with the catalytic action of the enzyme on a substrate, and provides an assay result determined by color development or color change upon action of the enzyme on the substrate, with a detection sensitivity at a nanogram level. Enzymes commonly used for labeling include horseradish peroxidase (HRP), alkaline phosphatase (AP), etc.

Enzyme-linked immunosorbent assay is extensively used in disease detection as no special instruments are required and the detection operation is simple. Common methods of the assay include an indirect method, a sandwich method and a BAS-ELISA method. The indirect method involves first coating a to-be-detected protein in a microplate, then adding a primary antibody, a second antibody labeled with an enzyme and a substrate sequentially and allowing a color to develop, and then quantitatively detecting the antigen by means of an instrument (such as a microplate reader). This method is simple in operation, but suffers from poor specificity due to a high background, and is now gradually replaced by the sandwich method. The sandwich method involves using two kinds of antibody to capture and immobilize a target antigen, and thus can greatly improve reaction specificity while ensuring sensitivity. An immunomagnetic capture method is a novel immunological technique that combines response capability of a magnetic bead to magnetic field with its immunospecificity. An immunomagnetic bead has the characteristics of a solid phase reagent and the advantages of high sensitivity, high specificity, etc., of an immunological reaction.

Additionally, there are also a serial cell counting and sorting technique based on the Coulter principle, which is capable of sorting micrometer-sized cells, and a nanopore DNA sequencing technique, which is mainly used in the analysis of nucleic acids. Both techniques are not yet used in the detection of proteins such as antigens.

## SUMMARY

The present application provides a method for detecting a trace protein in a sample system. In the method, an immunospecifically binding magnetic bead is used, a charge state of a single magnetic bead complex is detected, and an absolute amount of the trace protein is calculated. The method has a detection limit of down to a single protein molecule, can detect a trace protein at an amount below a lower limit of detection of conventional immunological detections, and can be widely used in immunological detection, microorganism detection, and cell separation, among other fields.

The present application is realized by the following technical solution:

A method for detecting a trace protein in a sample system, comprising:

providing a primary antibody-modified immunomagnetic bead and a secondary antibody-modified nanoparticle for a to-be-detected protein;

mixing and incubating the primary antibody-modified immunomagnetic bead and the secondary antibody-modified nanoparticle with a sample system containing the to-be-detected protein, so as to form a complex having a double-antibody sandwich structure; and

allowing the complex to pass through a micro-nano pore device in the form of a single particle and trigger an electrical pulse signal, analyzing the electrical pulse signal to obtain a charge state or a volume/mass state of the complex, and calculating, according to the charge state or the volume/mass state, the amount of the to-be-detected protein in the sample system.

In preferred embodiments, the above method further comprises: in a solution system that is suitable for an antibody modification reaction, allowing the primary antibody to covalently bind to a function group on a surface of the immunomagnetic bead to obtain the primary antibody-modified immunomagnetic bead.

In preferred embodiments, the degree of modification on the surface of the immunomagnetic bead by the primary antibody is adjusted by adjusting a concentration ratio of the primary antibody to the immunomagnetic bead in the solution system.

In preferred embodiments, the immunomagnetic bead has a particle size of at least 1 to 1000 times that of the nanoparticle.

In preferred embodiments, the immunomagnetic bead has a particle size of generally in the range of from 100 nm to 10 $\mu$m, and the nanoparticle has a particle size of generally smaller than 1 $\mu$m.

In preferred embodiments, magnetic particles are separated from excess unreacted nanoparticles by means of magnetic precipitation.

In preferred embodiments, the micro-nano pore device is a micro-nano pore single-particle counting device based on the Coulter principle. The device comprises two cavities filled with an electrolyte and a micro-nano pore that communicates the two electrolyte-filled cavities. When the complex passes through the micro-nano pore in the form of a single particle, it transiently blocks the flowing of ions through the micro-nano pore, thereby forming an electrical pulse signal.

In preferred embodiments, the electrical pulse signal reflects a charge state of the complex. The state and/or number of the nanoparticle bound on the complex is obtained by analyzing a peak fluctuation of the electrical pulse signal, and the amount of the to-be-detected protein in the sample system is calculated according to the state and/or number and a reaction principle of the double-antibody sandwich structure.

In preferred embodiments, the micro-nano pore device is a device having a micropore-bearing double-layer film structure. The device comprises two layers of micropore-bearing nano films spaced apart by a set distance. The micropores on the two nano films are disposed opposite to each other and communicate the cavities at non-opposing sides of the nano films. The cavities are filled with an electrolyte and are respectively provided with an electrode for maintaining ion transport. When the complex passes through the two micropores successively in the form of a single particle, a pair of electrical pulse signals having a time interval are generated.

In preferred embodiments, the method further comprises: adjusting a concentration of the complex in the sample such that the electrical pulse signal interval between respective complexes is much larger than the time interval between the pair of electrical pulse signals, and analyzing a pulse intensity threshold of the electrical pulse signals so as to filter off the signals generated by immunomagnetic beads not bound with the nanoparticle.

In preferred embodiments, with respect to the electrical pulse signal of the complex, an electromobility is calculated to obtain a particle surface potential of the complex.

In preferred embodiments, the electromobility is calculated according to the following formula:

$$\mu = \frac{v(x)}{E(x)} = \frac{\partial v(x)}{\partial E(x)}$$

where $\mu$ represents the electromobility, $v(x)$ represents a speed at which a complex particle passes through the two micropores successively, and $E(x)$ represents an electric field distribution;

The particle surface potential $\xi$ corresponds to the electromobility $\mu$ in the following relationship:

$$\mu = \frac{\epsilon \xi}{\eta}$$

where $\eta$ represents a viscosity of the electrolyte; and, the formula for the particle surface potential $\xi$ is as follows:

$$\xi = A \frac{\partial(\frac{1}{t})}{\partial V}$$

where $t$ represents a residence time of the particle in the micropores, $A$ represents a correction factor for each micropore, and V represents a potential difference.

In preferred embodiments, the volume/mass state is positively related to an integral area of the electrical pulse signal(s). The number of the protein molecules captured on the complex is directly proportional to an increase in volume/mass of the complex relative to the immunomagnetic bead. The number of the protein molecules captured on the complex is calculated according to a standard curve relationship of the integral area of the electrical pulse signal(s) and the number of the protein molecules captured on the complex, and in turn the amount of the to-be-detected protein in the sample system is calculated.

In the present application, an immunomagnetic bead is used to capture a to-be-detected trace protein by taking advantage of the characteristics of the immunomagnetic bead that it can be attracted by a magnetic field and its surface has a specific functional group capable of covalent binding with the biologically active protein. The immunomagnetic bead is used as a carrier of the to-be-detected protein. An antigen having an antigenic determinant (the to-be-detected protein) in a complex sample system, such as plasma, etc, is bound by the antibody coating the immunomagetic bead in an immunological reaction so that it is efficiently separated from the sample system, and it is recognized by the antibody labelling the nanoparticle, thereby achieving quantitative detection of the to-be-detected protein.

Moreover, in preferred embodiments, the degree of modification of the surface of the immunomagnetic bead by the antibody can be adjusted to achieve capture of one or more antigen protein molecules. The double-antibody sandwich structure formed facilitates increasing capture specificity, and the secondary antibody-connected nanoparticle can adjust the total charge of the complex having the double-antibody sandwich structure. Therefore, the method of the present application enables control over the number of protein molecules captured, which can be lower than 10 protein molecules/bead, and enables precise counting of the captured to-be-detected antigen protein by means of using the nanoparticle having a relatively small particle size.

## BRIEF DESCRIPTION OF DRAWINGS

Fig. 1 is a schematic depiction of the principle of a double-antibody sandwich structure for a single-protein complex in embodiments of the present application;

Fig. 2 is a schematic depiction of the principle of a double-antibody sandwich structure for a multiple-protein complex in embodiments of the present application;

Fig. 3 is a schematic depiction of a micro-nano pore single-particle counting device based on the Coulter principle in embodiments of the present application;

Fig 4. is a schematic depiction of the principle of determining a charge state of a complex particle by analyzing a minute fluctuation of an electrical pulse signal peak in embodiments of the present application;

Fig. 5 is a schematic depiction of a device having a micropore-bearing double-layer film structure in embodiments of the present application;

Fig. 6 is a schematic depiction of the principle of integrally forming the device having a double-layer film structure by means of micro-nano processing in embodiments of the present application;

Fig. 7 is a schematic depiction of a pair of electrical pulse signals generated when respective complexes pass through two micropores successively in the form of a single particle in embodiments of the present application;

Fig. 8 is a pulse diagram of true electrical pulse signal pairs when respective complexes pass through two micropores successively in the form of a single particle in embodiments of the present application;

Fig. 9 is a diagram showing a linear relationship of the number of captured protein molecules v.s. the electromobility (migration speed) in embodiments of the present application; and

Fig. 10 is a diagram showing electrical pulse signals (A) and a diagram showing a standard curve relationship of the integral area of the electrical pulse signals (the volume/mass of respective complexes) v.s. the number of captured protein molecules on the complexes.

## DESCRIPTION OF EMBODIMENTS

The present application will be further illustrated below by detailed description of embodiments with reference to the accompanying drawings. In the following embodiments, many details are described so that the present application will be better understood. However, those skilled in the art can readily recognize that some of the features may be omitted, or replaced by other materials or methods, depending on different situations.

Additionally, the characteristics, operations or features described in the specification can be combined in any suitable manner to form various embodiments. Moreover, the steps or actions in the description of the method may also be switched or adjusted in sequence in a manner that is obvious to those skilled in the art. Therefore, the various sequences in the description and the drawings are merely for the purpose of clearly describing a particular embodiment and are not intended to be required, unless it is otherwise specified that a specific sequence must be followed.

The present application provides a method for detecting a trace protein in a sample system.

In embodiments of the present application, the sample system can be a simple system containing a single protein component, or can be a complex system containing one or more proteins and at the same time containing other components, including blood, plasma, serum, tissue fluid, urine, cerebrospinal fluid, among other samples.

The method of the present application enables detection of a trace protein in a sample, for examples a protein present on a microgram, nanogram or picogram scale. The method has a minimum detection limit of down to a single protein molecule, can detect a trace protein at an amount below a lower limit of detection of conventional immunological detections, and can be widely used in immunological detection, microorganism detection, and cell separation, among other fields.

The method for detecting a trace protein in a sample system according to the present application comprises the following steps:

providing a primary antibody-modified immunomagnetic bead and a secondary antibody-modified nanoparticle for a to-be-detected protein;

mixing and incubating the primary antibody-modified immunomagnetic bead and the secondary antibody-modified nanoparticle with a sample system containing the to-be-detected protein, so as to form a complex having a double-antibody sandwich structure; and

allowing the complex to pass through a micro-nano pore device in the form of a single particle and trigger an electrical pulse signal, analyzing the electrical pulse signal to obtain a charge state or a volume/mass state of the complex, and calculating, according to the charge state or the volume/mass state, the amount of the to-be-detected protein in the sample system.

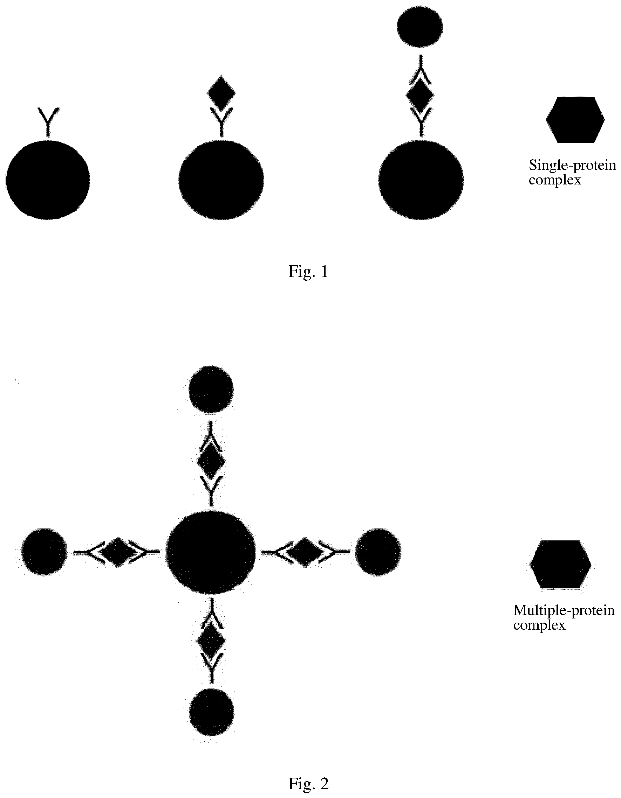

In embodiments of the present application, the primary antibody and the secondary antibody respectively bind to different antigenic determinants on the to-be-detected protein to form a double-antibody sandwich structure. As shown in Fig. 1, the Y-shaped structure represents an antibody, the larger sphere represents an immunomagnetic bead modified by a primary antibody on a surface thereof, the smaller sphere represents a nanoparticle modified by a secondary antibody on a surface thereof, the rhombus structure represents a to-be-detected protein, and the formed complex having a double-antibody sandwich structure can be structurally represented as immunomagnetic bead-primary antibody-to-be-detected protein-secondary antibody-nanoparticle. The complex having a double-antibody sandwich structure shown in Fig. 1 has only one to-be-detected protein (the rhombus structure in the figure), and thus can be termed as a "single-protein complex". The double-antibody sandwich structure according to the present application facilitates increasing capture specificity.

In embodiments of the present application, the primary antibody and the secondary antibody are not particularly limited in terms of the source and the type thereof, and can for example be a polyclonal antibody or a recombinantly expressed monoclonal antibody from various animal sources, such as an IgG antibody from a mouse, a goat, a rabbit, a horse, etc. In an embodiment of the present application, the primary antibody is mouse IgG, and the second antibody is a goat anti-mouse antibody.

In embodiments of the present application, a number of methods are available to modify the immunomagnetic bead with the primary antibody, which are not particularly limited in the present application.. In a preferred embodiment, in a solution system that is suitable for an antibody modification reaction, the primary antibody is allowed to covalently bind to a function group on a surface of the immunomagnetic bead to obtain the primary antibody-modified immunomagnetic bead. The functional group on the surface of the immunomagnetic bead can be an amino group, a carboxyl group, etc. and such an immunomagnetic bead can be termed as an amino magnetic bead, a carboxy magnetic bead, etc. The surface of the immunomagnetic bead is modified by the primary antibody by means of formation of covalent binding of the function group on the surface of the immunomagnetic bead with a corresponding group on the primary antibody. A typical reaction for modifying the immunomagnetic bead with the primary antibody is achieved under the action of EDC (1-ethyl-3-(3'-dimethylaminopropyl)carbodiimide) and NHS (N-hydroxysuccinimide). For example, in a preferred embodiment, the immunomagnetic bead (Ademtech) is exchanged with an MES buffer (2-(N-morpholinoethanesulfonic acid) for three times, and is equilibrated until the solution has a pH of 4-5 and an ionic strength of 0.1M. Into the equilibrated solution are added 1 mg of EDC and 1 mg of NHS, each dissolved in DMSO, and reaction is allowed for 20 minutes. Then the reaction mixture is centrifuged, and the supernatant is discarded. The precipitate obtaining after discarding the supernatant is redissolved using the MES buffer, and 0.1 mg of mouse IgG (Cat. :ab151276,Abcam) as the primary antibody is added and mixed to allow coating for 1 hour. Then BSA (Cat. :10735108001, Roche) as a terminating agent is added to a BSA concentration of 1% to terminate the reaction.

In preferred embodiments, the degree of modification of the surface of the immunomagnetic bead by the primary antibody is adjusted by adjusting a concentration ratio of the primary antibody to the immunomagnetic bead in the solution system. Single modification and multiple modification can be performed to adjust the number of secondary antibody-modified nanoparticles that can be bound, thereby forming a special complex. As shown in Fig. 2, the complex having a double-antibody sandwich structure comprises a plurality of to-be-detected protein molecules (represented by rhombus structures in the figure), and thus can also be termed as a "multiple-protein complex". For example, in a preferred embodiment, the concentration of the primary antibody is lower than 1 μg/mg immunomagnetic bead, such that the degree of modification of the surface of the immunomagnetic bead by the primary antibody is lower than 50 antibodies/immunomagnetic bead; in another preferred embodiment, the concentration of the primary antibody is 1-5 μg/mg immunomagnetic bead, such that the degree of modification of the surface of the immunomagnetic bead by the primary antibody is lower than 300 antibodies/immunomagnetic bead; and in yet another preferred embodiment, the concentration of the primary antibody is higher than 50 μg/mg immunomagnetic bead, such that the primary antibody is saturated on the immunomagnetic bead. In this way, the amount of primary antibody modification on the immunomagnetic bead can be controlled. The degree of modification can be further confirmed by electron microscopy.

In embodiments of the present application, the nanoparticle can be selected from nanoparticles of various materials and having various suitable particle sizes and various charge states, such as a polyethylene nano microsphere, a nano magnetic bead, a nano silicon sphere, a nano latex, etc. The particle size of the nanoparticle can for example be generally smaller than 1 micrometer, and in general, the corresponding immunomagnetic bead has a particle size that is at least greater than that of the nanoparticle, which enables achieving a good performance and facilitates formation of a special complex structure.

In embodiments of the present application, the principle and the method for preparing the secondary antibody-modified nanoparticle are similar to those for the primary antibody-modified immunomagnetic bead. A number of methods are available to modify the nanoparticle with the secondary antibody, which are not particularly limited in the present application. In a preferred embodiment, in a solution system that is suitable for an antibody modification reaction, the secondary antibody is allowed to covalently bind to a function group on a surface of the nanoparticle to obtain the secondary antibody-modified nanoparticle. As the nanoparticle carries a certain amount of charge, the number of the nanoparticle in the complex having a double-antibody sandwich structure formed according to the present application will influence the charge state of the entire complex. In the present application, it is by analyzing the charge state of the complex that the number of the nanoparticle on a single complex is obtained, and in turn the amount of the to-be-detected protein is analyzed and obtained.

In a typical embodiment of the present application, a reaction for modifying the nanoparticle with the secondary antibody is achieved under the action of EDC and NHS. For example, in a preferred embodiment, the secondary antibody is a goat anti-mouse antibody, and the preparation method for the nanoparticle (Cat. :F122270, aladdin) labelled with the goat anti-mouse antibody comprises the following steps: equilibrating 1 mg of the nanoparticle in a PBS solution having a pH of 8.0 and a concentration of 0.1 M to a final concentration of the nanoparticle of 0.1 mg/mL; then adding EDC and NHS until a final concentration of each of EDC and NHS in the solution of 0.2 mg/mL, allowing activation for 30 minutes, centrifuging the reaction mixture and discarding the supernatant, redissoving the precipitate obtained after discarding the supernatant in a PBS solution, adding a goat anti-mouse antibody (Cat. :4T20, HyteSt) and allowing labelling for 25 minutes, and adding BSA until a concentration of 0.5 % to terminate the reaction.

The protein detection method according to the present application is performed based on an electrical pulse signal, which is triggered when the complex having a double-antibody sandwich structure formed during the immunological reaction undergoes electrophoresis in the electrolyte and passes through the micropore(s). A charge state of the complex can be obtained by analyzing the electrical pulse signal, and the amount of the to-be-detected protein in the sample system can be calculated according to the charge state. In an embodiment of the present application, the complex having a double-antibody sandwich structure passes through a micro-nano pore device in the form of a single particle and triggers an electrical pulse signal, which can be analyzed to obtain a charge state of the complex, which in turn can be used to calculate the amount of the to-be-detection protein in the sample system.

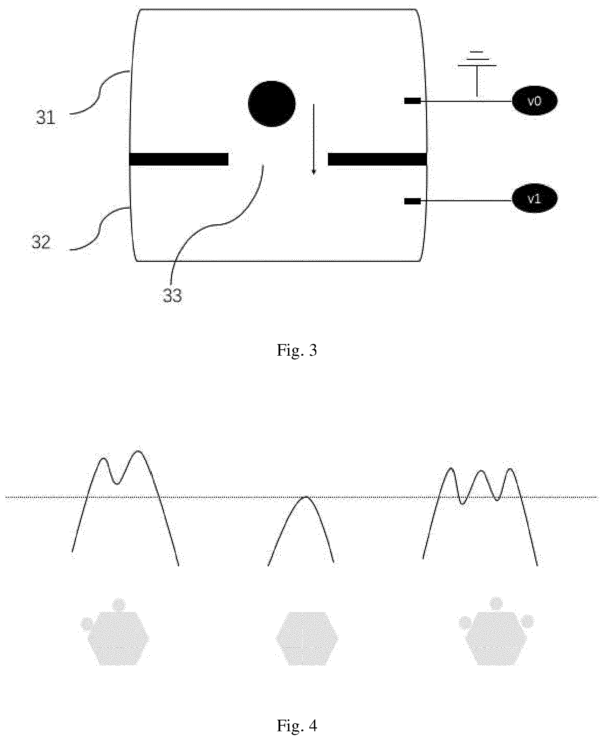

A number of methods are available to implement the protein detection method based on an electrical pulse signal according to the present application. One method involves a micro-nano pore single-particle counting device based on the Coulter principle (for example, US patent No. 2,656,508, 1953), which detects signals of respective complexes in the micro-nano pore detection device. As shown in Fig. 3, the micro-nano pore single-particle counting device comprises two electrolyte-filled cavities, that is, cavity 31 and cavity 32 in the figure, and a sole micro-nano pore 33 that communicates the two electrolyte-filled cavities. When the complex passes through the micro-nano pore 33 in the form of a single particle under a driving force (such as, an electric field, a pressure, an electroosmotic flow, etc.), it transiently blocks the flowing of ions through the micro-nano pore 33, thereby forming an electrical pulse signal. The electrical pulse signal can reflect ion size, binding state, charge state and even geometry. The binding state (whether bound or not) of the nanoparticle and the binding number of the nanoparticle in the complex is obtained by analyzing a peak fluctuation of the electrical pulse signal, and the amount of the to-be-detected protein in the sample system is calculated according to the binding state and/or number of the nanoparticle and a reaction principle of the double-antibody sandwich structure. As shown in Fig. 4, in an embodiment of the present application, a threshold of the intensity of the electrical pulse signal can be analyzed to determine a charge state of the complex particle, and a minute fluctuation of the electrical pulse signal peak can be analyzed to determine whether the nanoparticle is bound on the complex particle, as well as the binding number. For example, in Fig. 4, when two nanoparticles are bound on the complex particle, the electrical pulse signal is reflected by two peaks; when no nanoparticle is bound on the complex particle, the electrical pulse signal is reflected by absence of peak; and when three nanoparticles are bound on the complex particle, the electrical pulse signal is reflected by three peaks.

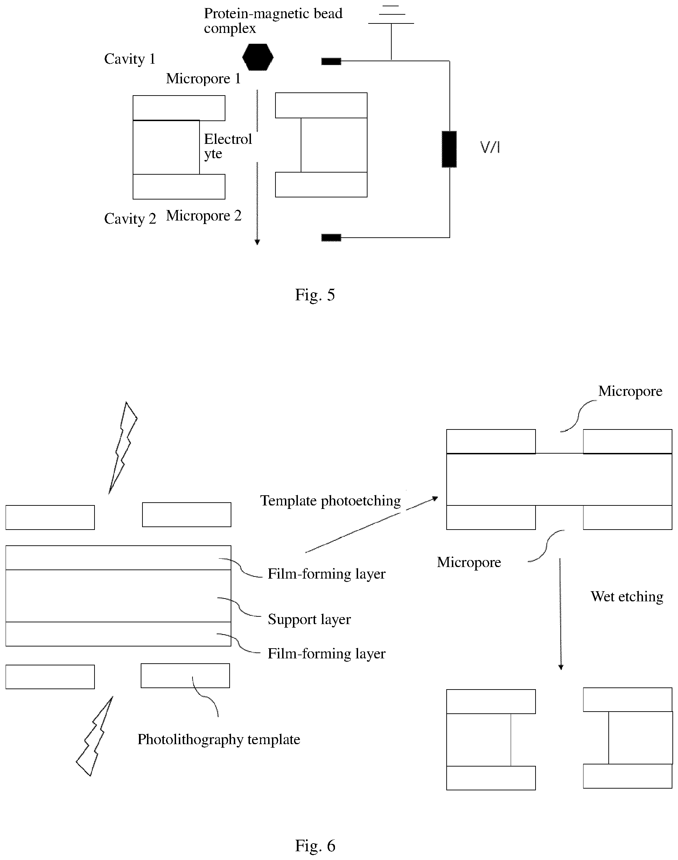

In another embodiment of the present application, the micro-nano pore device is a device having a micropore-bearing double-layer film structure. As shown in Fig. 5, the device comprises two layers of micropore-bearing nano film (micropore 1 and micropore 2 in the figure) spaced apart by a set distance. The micropores on the two nano films are disposed opposite to each other and communicate the cavities (cavity 1 and cavity 2 in the figure) at non-opposing sides of the nano films. The cavities are filled with an electrolyte and are respectively provided with an electrode for maintaining ion transport. When the protein-magnetic bead complex passes through the two micropores successively in the form of a single particle, a pair of electrical pulse signals having a time interval are generated.

In the above embodiment, the size of the micropores in the device having a double-layer film structure can be in the range from 1 nm to 10 $\mu$m, and can be specifically determined according to the requirement of allowing the protein-magnetic bead complex to pass through the micropores successively in the form of a single particle. The nano film can be selected from films prepared from any semiconductor film-forming materials such as silicon nitride, silicon dioxide, silicon, etc., or from high-molecular polymers. The two layers of nano film are spaced apart by a set distance, which can be from 1 nm to 1 $\mu$m. The device having a double-layer film structure is equipped with two electrodes, which can be a silver/silver chloride electrode or a platinum electrode. An external electric field is applied to maintain a balanced ion transport, which is reflected by a stable open pore current. When the protein-magnetic bead complex passes through the two micropores successively under the action of the driving force, a pair of electrical pulse signals having a time interval are generated.

The device having a double-layer film structure according to the present application can be obtained by various suitable methods. For example, in an embodiment of the present application, the device having a double-layer film structure is integrally formed by means of micro-nano processing to control the distance of the two layers of film. The specific principle and steps are as shown in Fig. 6. In step one, a substrate material (silicon, quartz, etc.) having been polished on both sides thereof is provided as a support layer, and a nano film (film-forming layer in the figure) of 1 nm to 10 $\mu$m is prepared on the upper surface and the lower surface of the substrate material by means of thermal oxidation or vapor deposition. In step two, previously-prepared photolithography templates are used to define the size and shape of the micropores and the position thereof on the films. A photoresist is coated on the upper and lower surfaces, and the templates are used to perform light exposure and development. The upper and lower templates can be precisely aligned to ensure that the two micropores maintain a micron-scale alignment precision in space. Then, with the unexposed photoresist as a sacrificial layer, the films are etched by means of gas etching to form the micropores. In step three, the support layer is etched by means of wet etching in which an etching solution etches the support layer from the micropores so that the two micropores are in communication with each other.

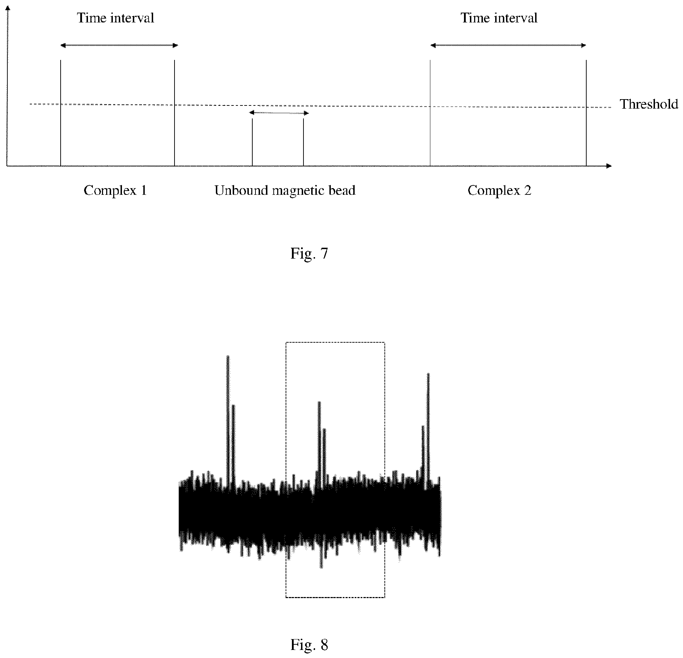

As shown in Fig. 7, when complex 1, an unbound magnetic bead (i.e., an immunomagnetic bead not bound with the nanoparticle), and complex 2 pass through the two micropores successively in the form of a single particle, a pair of electrical pulse signals for each of them are generated. The electrical pulse signals have a certain time interval, which reflects the time for passing through the micropores and is associated with electromobility, which in turn is associated with charge state. The time interval between the electrical pulse signals for complex 1 and complex 2 exceeds a set threshold, while the time interval between the electrical pulse signals for the unbound magnetic bead does not exceed the set threshold. Fig. 8 shows true pairs of electrical pulse signals generated when respective complexes pass through the two micropores successively in the form of a single particle. It can be seen that a pair of electrical pulse signals is generated when each complex particle passes through the micropores, and the two electrical pulse signals of the pair have a certain time interval.

In a preferred embodiment of the present application, the concentration of the complex in the sample is adjusted (for example, smaller than 10 picogram/milliliter) such that the electrical pulse signal interval between respective complexes is much larger than the time interval between the pair of electrical pulse signals, and a pulse intensity threshold of the electrical pulse signals is analyzed so as to filter off the signals generated by immunomagnetic beads not bound with the nanoparticle.

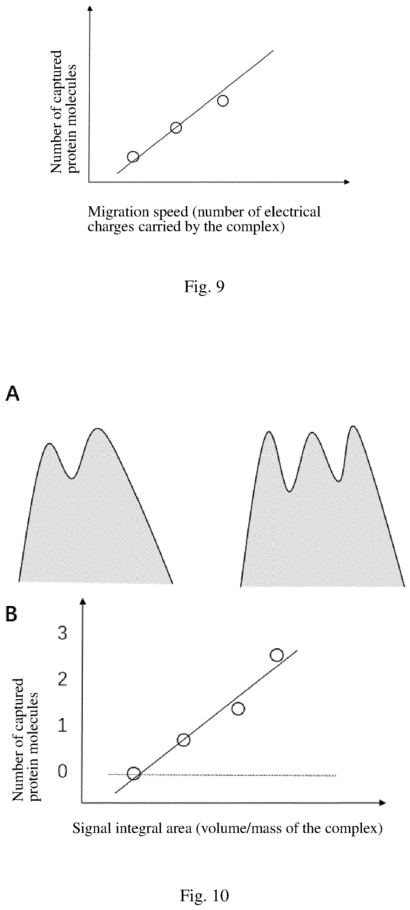

According to a reaction principle of the double-antibody sandwich structure of the present application, the number of protein molecules captured on the immunomagnetic bead determines the number of the secondary antibody-modified nanoparticle bound thereto, and in turn determines the number of electrical charges carried by the complex, which is associated with electromobility. Therefore, as shown in Fig. 9, a linear relationship exists between the number of captured protein molecules and the electromobility (migration speed). A particle surface potential can be obtained by calculating the electromobility, and then the amount of the to-be-detected protein can be calculated.

In an embodiment of the present application, the electromobility is calculated according to the following formula:

$$\mu = \frac{v(x)}{E(x)} = \frac{\partial v(x)}{\partial E(x)}$$

where $\mu$ represents the electromobility, $v(x)$ represents a speed at which a complex particle passes through the two micropores successively, and $E(x)$ represents an electric field distribution.

For example, suppose that a distance of the two micropores of 1000 nm, a time interval of 1 millisecond during which a single complex particle passes through the two micropores, and a potential difference of 100 millivolts, then the electromobility can be calculated to be $10^{-8} m^2 V^{-1} s^{-1}$.

Moreover, the particle surface potential $\xi$ corresponds to the electromobility $\mu$ in the following relationship:

$$\mu = \frac{\epsilon \xi}{\eta}$$

where $\eta$ represents a viscosity of the electrolyte. and, the formula for the particle surface potential $\xi$ is as follows:

$$\xi = A \frac{\partial(\frac{1}{t})}{\partial V}$$

where $t$ represents a residence time of the particle in the micropores, $A$ represents a correction factor for each micropore, and V represents a potential difference.

For example, in an embodiment of the present application, suppose that the correction factor A is $6 \times 10^{-6}$ $V^2$ s, then the particle surface potential $\xi$ can be calculated to be 60 millivolts.

In another embodiment of the present application, the amount of the to-be-detected protein in a sample system can be calculated according to the volume/mass state of the complex. In particular, as shown in Fig. 10, the volume/mass state of the complex is positively related to an integral area of the electrical pulse signal. The number of the protein molecules captured on the complex is directly proportional to an increase in volume/mass of the complex relative to the immunomagnetic bead. The number of the protein molecules captured on the complex is be calculated according to a standard curve relationship of the integral area of the electrical pulse signal (which characterizes the volume/mass state of the complex) and the number of the protein molecules captured on the complex, and in turn the amount of the to-be-detected protein in the sample system is calculated. In this embodiment, the volume/mass of the complex represents the volume, the mass, or a combination of both of the complex, because according to a reaction principle of the double-antibody sandwich structure of the present application, the number of protein molecules captured on the immunomagnetic bead determines the number of the secondary antibody-modified nanoparticle bound thereto, and latter in turn determines an increase in the volume or mass of the complex.

The present application has been described above with reference to specific embodiments, which are merely intended to aid in the understanding of the present application and are not intended to limit the present application thereto. Several simple derivations, variations or substitutions can be made by a person skilled in the art to which the present application pertains in light of the concept of the present application.

**Claims**

1. A method for detecting a trace protein in a sample system, the method comprising:

   providing a primary antibody-modified immunomagnetic bead and a secondary antibody-modified nanoparticle for a to-be-detected protein;
   mixing and incubating the primary antibody-modified immunomagnetic bead and the secondary antibody-modified nanoparticle with a sample system containing the to-be-detected protein, so as to form a complex having a double-antibody sandwich structure; and
   allowing the complex to pass through a micro-nano pore device in the form of a single particle and trigger an electrical pulse signal, analyzing the electrical pulse signal to obtain a charge state or a volume/mass state of the complex, and calculating, according to the charge state or the volume/mass state, the amount of the to-be-detected protein in the sample system.

2. The detection method according to claim 1, wherein the method further comprises: in a solution system that is suitable for an antibody modification reaction, allowing the primary antibody to covalently bind to a function group on a surface of the immunomagnetic bead to obtain the primary antibody-modified immunomagnetic bead.

3. The detection method according to claim 2, wherein the degree of modification on the surface of the immunomagnetic bead by the primary antibody is adjusted by adjusting a concentration ratio of the primary antibody to the immunomagnetic bead in the solution system.

4. The detection method according to claim 1, wherein the immunomagnetic bead has a particle size of at least 1 to 1000 times that of the nanoparticle; preferably, the immunomagnetic bead has a particle size of generally in the range of from 100 nm to 10 $\mu$m, and the nanoparticle has a particle size of generally smaller than 1 $\mu$m.

5. The detection method according to claim 1, wherein the micro-nano pore device is a micro-nano pore single-particle counting device based on the Coulter principle; the device comprises two cavities filled with an electrolyte and a micro-nano pore that communicates the two electrolyte-filled cavities; when the complex passes through the micro-nano pore in the form of a single particle, it transiently blocks the flowing of ions through the micro-nano pore, thereby forming an electrical pulse signal.

6. The detection method according to claim 5, wherein the electrical pulse signal reflects a charge state of the complex; the state and/or number of the nanoparticle bound on the complex is obtained by analyzing a peak fluctuation of the electrical pulse signal, and the amount of the to-be-detected protein in the sample system is calculated according to the state and/or number and a reaction principle of the double-antibody sandwich structure.

7. The detection method according to claim 1, wherein the micro-nano pore device is a device having a micropore-bearing double-layer film structure, the device comprises two layers of micropore-bearing nano films spaced apart by a set distance, the micropores on the two nano films are disposed opposite to each other and communicate the cavities at non-opposing sides of the nano films, and the cavities are filled with an electrolyte and are respectively provided with an electrode for maintaining ion transport; when the complex passes through the two micropores successively in the form of a single particle, a pair of electrical pulse signals having a time interval are generated.

8. The detection method according to claim 7, wherein the method further comprises: adjusting a concentration of the complex in the sample such that the electrical pulse signal interval between respective complexes is much larger than the time interval between the pair of electrical pulse signals, and analyzing a pulse intensity threshold of the electrical pulse signals so as to filter off the signals generated by immunomagnetic beads not bound with the nanoparticle.

9.  The detection method according to claim 8, wherein with respect to the electrical pulse signal of the complex, an electromobility is calculated to obtain a particle surface potential of the complex;

    preferably, the electromobility is calculated according to the following formula:

    $$\mu = \frac{v(x)}{E(x)} = \frac{\partial v(x)}{\partial E(x)}$$

    where $\mu$ represents the electromobility, $v(x)$ represents a speed at which a complex particle passes through the two micropores successively, and $E(x)$ represents an electric field distribution;
    the particle surface potential $\xi$ corresponds to the electromobility $\mu$ in the following relationship:

    $$\mu = \frac{\epsilon \xi}{\eta}$$

    where $\eta$ represents a viscosity of the electrolyte;
    and, the formula for the particle surface potential $\xi$ is as follows:

    $$\xi = A \frac{\partial(\frac{1}{t})}{\partial V}$$

    where $t$ represents a residence time of the particle in the micropores, $A$ represents a correction factor for each micropore, and $V$ represents a potential difference.

10. The detection method according to claim 1, wherein the volume/mass state is positively related to an integral area of the electrical pulse signal(s), the number of the protein molecules captured on the complex is directly proportional to an increase in volume/mass of the complex relative to the immunomagnetic bead, the number of the protein molecules captured on the complex is calculated according to a standard curve relationship of the integral area of the electrical pulse signal(s) and the number of the protein molecules captured on the complex, and in turn the amount of the to-be-detected protein in the sample system is calculated.

Single-protein complex

Fig. 1

Multiple-protein complex

Fig. 2

31

32

33

v0

v1

Fig. 3

Fig. 4

Protein-magnetic bead complex

Cavity 1

Micropore 1

Electrolyte

Cavity 2   Micropore 2

V/I

Fig. 5

Template photoetching

Micropore

Micropore

Film-forming layer

Support layer

Film-forming layer

Photolithography template

Wet etching

Fig. 6

Fig. 7

Fig. 8

Migration speed (number of electrical
charges carried by the complex)

Fig. 9

**A**

**B**

Signal integral area (volume/mass of the complex)

Fig. 10

## INTERNATIONAL SEARCH REPORT

| International application No. |
|---|
| **PCT/CN2020/128990** |

### A. CLASSIFICATION OF SUBJECT MATTER

G01N 33/543(2006.01)i;   G01N 33/68(2006.01)i

According to International Patent Classification (IPC) or to both national classification and IPC

### B. FIELDS SEARCHED

Minimum documentation searched (classification system followed by classification symbols)

G01N33 G01N15 C12Q1

Documentation searched other than minimum documentation to the extent that such documents are included in the fields searched

Electronic data base consulted during the international search (name of data base and, where practicable, search terms used)

CNABS; CNTXT; CNKI; 百度学术; 万方: 抗体, 一抗, 二抗, 磁, 脉冲, 微纳孔, 纳米孔, 迁移率, 脉冲对, 球, 粒, 珠 EPTXT; USTXT; VEN; WOTXT; JPTXT; Web of science: antibody, magnetic, pulse, nanopore, second, two, dual, sphere, bead, particle

### C. DOCUMENTS CONSIDERED TO BE RELEVANT

| Category* | Citation of document, with indication, where appropriate, of the relevant passages | Relevant to claim No. |
|---|---|---|
| X | EP 2573554 A1 (NXP BV.) 27 March 2013 (2013-03-27)<br>  claims 1, 13, description, paragraphs 2-4, embodiment 13, figure 9 | 1-6, 10 |
| Y | EP 2573554 A1 (NXP BV.) 27 March 2013 (2013-03-27)<br>  claims 1, 13, description, paragraphs 2-4, embodiment 13, figure 9 | 7-9 |
| Y | EP 2311975 A1 (IEE SARL) 20 April 2011 (2011-04-20)<br>  claims1, description, paragraphs 6-7, 32-33, figure 5 - figure 10 | 7-9 |
| PX | CN 111830251 A (RUIXIN ZHIZAO (SHENZHEN) TECHNOLOGY CO., LTD.) 27 October 2020 (2020-10-27)<br>  description, specific embodiments, figures 1-4 | 1-6, 10 |
| A | WO 2016187159 A2 (TWO PORE GUYS INC.) 24 November 2016 (2016-11-24)<br>  entire document | 1-10 |
| A | CN 108344863 A (CHONGQING INSTITUTE OF GREEN AND INTELLIGENT TECHNOLOGY, CHINESE ACADEMY OF SCIENCES) 31 July 2018 (2018-07-31)<br>  entire document | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 January 2021** | **18 February 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/128990**

| Patent document cited in search report | | | Publication date (day/month/year) | Patent family member(s) | | | Publication date (day/month/year) |
|---|---|---|---|---|---|---|---|
| EP | 2573554 | A1 | 27 March 2013 | None | | | |
| EP | 2311975 | A1 | 20 April 2011 | None | | | |
| CN | 111830251 | A | 27 October 2020 | None | | | |
| WO | 2016187159 | A2 | 24 November 2016 | EP | 3295161 | A2 | 21 March 2018 |
| | | | | US | 2018363035 | A1 | 20 December 2018 |
| CN | 108344863 | A | 31 July 2018 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2656508 A